# EUROPÄISCHE PATENTANMELDUNG

(11) **EP 2 093 279 A2**
(43) Veröffentlichungstag der Anmeldung: **26.08.2009**
(21) Anmeldenummer: 09002402.7
(22) Anmeldetag: 20.02.2009
(51) Int. Cl.: C12M 1/42

(54) **Zellkultur-Bestrahlungskammer**

(30) Priorität: 25.02.2008 DE 102008010918
(71) Anmelder: GSI Helmholtzzentrum für Schwerionenforschung GmbH, 64291 Darmstadt (DE)
(72) Erfinder: Schicker, Corinna, 64347 Griesheim (DE); Kopf, Ulrich, 64347 Griesheim (DE); Kraf-Weyrather, Wilma, 64291 Darmstadt (DE); Von Neubeck, Cläre Hanna Freiin, 64285 Darmstadt (DE)
(74) Vertreter: Mergel, Volker

(57) **Zusammenfassung**

Die Erfindung betrifft eine Zellkultur-Bestrahlungskammer (10) zur Bestrahlung von lebenden Zellen mit einem Röntgenstrahl oder Teilchenstrahl (52) zum experimentellen Ermitteln der Überlebensrate von Testzellen in der Tumorstrahlentherapie u.a. unter hypoxischen Bedingungen. Die Zellkultur-Bestrahlungskammer umfasst einen Trägerrahmen (12) mit beidseits lösbar aufgeklebten gaspermeablen Folien (50a,50b), um ein geschlossenen Zellkulturvolumen (16) zu definieren. Zur Steuerung der Bedingungen wird die Zellkultur-Bestrahlungskammer vor der Bestrahlung in einem speziellen Begasungscontainer (30) begast, in welchem auch die Bestrahlung stattfindet.

## Beschreibung

### Gebiet der Erfindung

Die Erfindung betrifft eine Zellkultur-Bestrahlungskammer zur Bestrahlung von lebenden Zellen im Allgemeinen und zur Bestrahlung von hypoxischen adhärent wachsenden Zellen im Speziellen sowie ein Bestrahlungssystem mit einem Begasungscontainer und ein Verfahren zur Bestrahlung der Zellen mit dem Bestrahlungssystem.

### Hintergrund der Erfindung

In der Tumorbehandlung hat sich seit vielen Jahrzehnten die Bestrahlung mit Röntgenstrahlen und für spezielle Tumore auch mit Elektronenstrahlen bewährt. Neuerdings werden bestimmte Tumorarten allerdings auch mit hochenergetischen Ionen bestrahlt. So wurde an der Gesellschaft für Schwerionenforschung GmbH (GSI) eine Bestrahlungstherapie mit Kohlenstoffionen entwickelt. Sie kombiniert eine tumorkonforme Strahlenapplikation mit einer biologisch optimierten Bestrahlungsplanung. Seit 1997 wurden an der GSI ca. 400 Patienten, vorwiegend mit Tumoren im Hals-KopfBereich, bestrahlt und gute Heilungserfolge erzielt.

Es ist bekannt, dass hypoxische Zellen eine erhöhte Strahlenresistenz aufweisen. Hypoxische Tumorareale entstehen, wenn die Gefäßbildung (Transport von Gas, Nährstoffen etc.) dem Tumorwachstum nicht folgen kann. Dabei versteht man unter Hypoxie einen geringeren Sauerstoffgehalt in den und um die Zellen als bei normal durch die Gefäße versorgten Zellen. Der Begriff "Hypoxie" wird als Bezeichnung für den gesamten Konzentrationsbereich zwischen geringerem Sauerstoffgehalt als normal und Anoxie (Abwesenheit von Sauerstoff) verwendet.

Die experimentelle Untersuchung der Zusammenhänge von Überlebenswahrscheinlichkeit, Sauerstoffgehalt der Zellen und Strahlungsdosis ist von großem Interesse in der weiteren Erforschung und Anwendung der TumorStrahlentherapie. Um derartige Untersuchungen vornehmen zu können, werden Testzellen kultiviert und unter hypoxischen Bedingungen untersucht.

Aus Hirayama et al. "Repair Kinetics of DNA-DSB Induced by X-Rays or Carbon Ions under Oxic and Hypoxic Conditions", Journal of Radiation Research (2005), 46:325-332, ist es bekannt, CHO-Zellen in Glas-Petrischalen zu kultivieren. Diese werden dann in eine Bestrahlungskammer überführt. Die Kammer wird für die Bestrahlung unter oxischen Bedingungen mit einem Gasgemisch aus 5 % Kohlendioxid und Luft gefüllt. Für die Bestrahlung unter hypoxischen Bedingungen wird die Kammer für mindestens eine Stunde mit einem Gasgemisch aus 95 % Stickstoff und 5 % CO₂ begast. Die Begasung wird auch während der Bestrahlung mit einer Flussrate von 200 ml/min fortgeführt. Die Zellen wurden wahlweise mit Röntgenstrahlung (4,9 Gy/min, 200 kV, 20 mA) oder Kohlenstoffionen (linearer Energietransfer (LET): 80 keV/µm) bestrahlt. Das Überleben der Zellen wurde mit einem Klonogenitätstest bestimmt.

Dieses Verfahren eignet sich aber nur bedingt für die Simulation hypoxischer Tumore. In nachteiliger Weise wird einerseits bei der Bestrahlung von Glas durch Rückstreuung die Dosis auf die Zellen verändert. Andererseits erlauben die relativ dicken Glasschichten auch nicht die Bestrahlung von mehreren Zellkulturen hintereinander, da der Strahl die Glasschichten nicht durchdringt. Ferner ist das Verfahren aufwändig und die Genauigkeit der Bestimmung des Sauerstoffgehaltes in den Zellen ist verbesserungswürdig.

Aus Schmaltz et al. "Regulation of Proliferation-Survival Decisions During Tumor Cell Hypoxia", Molecular and Cellular Biology (1998), 18:2845-2854, ist bekannt, Zellen auf Polystyrol zu kultivieren. Hierzu wurden Fibroplasten auf Permanox-Petrischalen, deren Boden aus einer gasdurchlässigen Folie bestand, kultiviert. Die Mediumschicht in diesen Zellkulturgefäßen war gering, um einen raschen Gasaustausch zu ermöglichen. Die Hypoxie in den Zellkulturen wurde auf zwei verschiedene Arten erreicht: Entweder wurden die Petrischalen in Aluminium-Hypoxiekammern gelegt, die in einem Brutschrank mit einer Gasmischung aus 5 % CO₂ und 95 % N₂ begast wurden oder es wurden die Zellkulturgefäße in eine Anaerobie-Kammer mit integrierter Temperiereinheit gestellt. Für diese Versuche wurden kommerziell erhältliche Hypoxie-Kammern von Billups-Rothenberg, Delmar, USA, mit einer runden Kunststoffhaube, die mit einem Metallring auf einem Kunststoffboden befestigt ist, verwendet. Die Zellkulturgefäße wurden in die Kammern eingestellt. Die Begasung erfolgte dann über die Haube. Eine Bestrahlung der hypoxischen Zellen mit einem Ionenstrahl war hier nicht vorgesehen und wäre auch nicht oder nur sehr schwierig möglich.

Die DE 100 32 808 A1 beschreibt Zellkulturkammern für einen Bioreaktor, die übereinander gestapelt werden, um eine vordefinierte Formgebung von Gewebe bzw. Organen zu erzielen. Hierzu werden mehrere flache Kammern multilayerartig übereinander gestapelt, wobei die Membranen der einzelnen Kammern resorbierbar sein sollen, um ein drei-dimensional beliebig formbares Gewebe, z.B. ein dreischichtiges Hautgewebe ggf. ein ganzes Organ, zu züchten. Die Membranen liegen unmittelbar aufeinander und Gase werden durch Hohlfasermembranen eingeleitet. Die Vorrichtung dient der kommerziellen Produktion eines Zellkompartiments mit unterschiedlichen Zelllinien (allen drei Hautschichten) als transplantierbarem Gewebe als Endprodukt, besitzt keine gaspermeablen Membranen und scheint nicht für eine Bestrahlung geeignet zu sein.

Die DE 20 2006 012 978 U1 beschreibt einen Bioreaktor, bestehend aus einer Basisplatte mit einer Aussparung, welche beidseits von transparenten Deckelfolien abgedeckt wird. Bei den Deckelfolien geht es darum, die Zellen besser mikroskopieren zu können, weshalb auf die Transparenz der Deckelfolien besonderen Wert gelegt wird. Daher werden Deckelfolien mit bestimmten Brechungsindizes verwendet, z.B. Cycloolefincopolymer (COC) oder Cycloolefinpolymer (COP). Die Basisplatte weist Anschlüsse in der oberen transparenten Deckelfolie auf. Diese Anschlüsse ermöglichen die aktive Versorgung der Zellen in Form einer Perfusion oder Superfusion. Somit werden die Bedingungen in dem Bioreaktor offenbar über die fluidischen Anschlüsse eingestellt. Gastransport durch die Deckelfolien ist nicht von Interesse.

Die US 2005/0112758 A1 beschreibt einen Transportbehälter für tumoröses Gewebe nach einer Operation, z.B. für die pathologische Untersuchung, um das Personal, welches mit dem tumorösen Geweben umgeht, vor Kontakt bzw. Kontamination zu schützen. Besonders wichtig hierbei ist also die Dichtigkeit der Vorrichtung. Um das Personal vor Kontakt mit dem Gewebe zu schützen, wird ein flexibler Abschnitt zum Eingreifen und Drehen der Gewebeprobe durch den Benutzer vorgeschlagen.

Aus der WO 01/09282 ist eine rahmenartige Trägerplatte bekannt, jedoch wird diese rahmenartige Trägerplatte mit einer Abdeckplatte verwendet. Diese Anordnung ist offensichtlich nicht für die Bestrahlung der Zellkulturen vorgesehen oder geeignet, da kein Fenster vorhanden ist. Es geht lediglich um das Züchten von Zellen.

Die DE 10 2006 018 824 A1 beschreibt einen Einweg-Bioreaktor, bei dem es um den rotierenden Antrieb für ein gutes Mischen geht. Dieser Bioreaktor ist jedoch nicht flach mit einer rahmenartigen Trägerplatte, sondern z.B. rund oder viereckig mit entsprechend großem Volumen. Der Reaktor eignet sich nicht zur Bestrahlung oder um definierte hypoxische Bedingungen für Zellen einzustellen.

Aus der US 5,843,766 ist eine Gewebekulturkammer und aus der US 2003/0032174 A1 ist ein Gerät zum Züchten von Pflanzen bekannt.

### Allgemeine Beschreibung der Erfindung

Daher ist es eine Aufgabe der Erfindung, ein Zellkulturbehältnis bereit zu stellen, welches sich zur Kultivierung von lebenden Zellen und für die Bestrahlung mit einem Röntgenstrahl und einem Teilchenstrahl eignet, also auch mit einem horizontalen Strahl, und das kostengünstig herstellbar und zumindest teilweise wiederverwertbar ist.

Eine weitere Aufgabe ist es, eine Vorrichtung und ein Verfahren bereit zu stellen, mit denen das Verhalten von Zellen, die durch Hypoxie eine erhöhte Strahlenresistenz aufweisen, untersucht werden kann, z.B. um experimentelle Daten zu gewinnen, die in die Bestrahlungsplanung der Tumortherapie integriert werden können. Hierzu soll ein definierter Sauerstoffgehalt an der Zellschicht eingestellt werden und diese Zellen sollen unter definierten Begasungsbedingungen bestrahlbar sein, wobei unerwünschte Störeffekte so weit wie möglich vermieden werden sollen.

Erfindungsgemäß wird eine Zellkultur-Bestrahlungskammer zur Bestrahlung von lebenden Zellen mit einem Röntgenstrahl oder Teilchenstrahl bereitgestellt. In der Zellkultur-Bestrahlungskammer werden Testzellen kultiviert und mit dem Röntgenstrahl oder Teilchenstrahl bestrahlt, um das Verhalten der Testzellen auf die Bestrahlung unter vordefinierten Bedingungen zu untersuchen, um hiermit wissenschaftliche Rückschlüsse auf das Verhalten der Tumorzellen vor, während und nach der Bestrahlung zu ziehen. Besonderes Augenmerk wird hierbei auf die experimentelle Ermittlung der Überlebensrate der Testzellen unter hypoxischen Bedingungen, d.h. unter Sauerstoffmangel gelegt. Der Teilchenstrahl kann z.B. ein Ionenstrahl aus Kohlenstoffionen sein, wie er für die Tumortherapie bei der Gesellschaft für Schwerionenforschung mbH in Darmstadt und dem Deutschen Krebsforschungszentrum in Heidelberg verwendet wird. Die Zellkultur-Bestrahlungskammer eignet sich aber auch für die Untersuchung des Verhaltens von Testzellen in der konventionellen Röntgentumortherapie oder andere Bestrahlungsexperimente.

Die Zellkultur-Bestrahlungskammer weist einen Trägerrahmen auf, der einen Innenraum umschließt. Zweckmäßig besteht der Trägerrahmen aus einer flachen Trägerplatte, welche eine relativ große Bohrung quer zu der Plattenebene aufweist, so dass die Trägerplatte einen Ring mit einer umlaufenden Ringwandung um den Innenraum bildet, so dass die innere Ringwandung den Innenraum lateral begrenzt. Ein solcher Ring, insbesondere aus Kunststoff, z.B. PVC ist einfach und kostengünstig herzustellen und gut zu reinigen bzw. zu sterilisieren.

Auf beiden Seiten des Trägerrahmens sind Folien aufgebracht, die die Bohrung vollständig überspannen und flüssigkeitsdicht befestigt, z. B. aufgeklebt, sind, so dass der Innenraum beidseits von den Folien flüssigkeitsdicht verschlossen wird und eine im Wesentlichen geschlossene Zellkultur-Bestrahlungskammer, begrenzt durch die beiden Folien und die innere Ringwandung, gebildet wird. Dieser Innenraum bildet das Volumen in dem die Zellen kultiviert und bestrahlt werden und wird daher als Zellkulturvolumen bezeichnet. Im Wesentlichen geschlossen heißt in diesem Zusammenhang nicht, dass die Zellkultur-Bestrahlungskammer vollständig geschlossen sein muss, da wie nachfolgend erläutert wird, noch eine verschließbare radiale Bohrung in dem Trägerrahmen zum Befüllen des Zellkulturvolumens vorgesehen sein kann. Mit anderen Worten werden die beiden Folien von dem flächigen Trägerrahmen oder Ring parallel beabstandet gehalten und zwischen den beiden Folien wird ein mit einem Zellkultur- oder Nährmedium befüllbares Zellkulturvolumen gebildet, welches beidseits unmittelbar von den beiden Folien begrenzt wird, so dass das Nährmedium oder dies Nährlösung beiderseits in unmittelbarem Kontakt mit den Folien steht.

Zumindest eine der beiden Folien, vorzugsweise beide Folien, sind gaspermeabel. Die Folien bilden also eine gaspermeable Membran die unmittelbar die Umgebungsatmosphäre von dem Zellkulturvolumen mit dem flüssigen. Nährmedium und den Zellen trennt. Somit können Gasmoleküle durch die gaspermeable Folie in das Zellkulturvolumen hinein- und herausdiffundieren. Dadurch kann der Gehalt vorbestimmter Gasmoleküle in den lebenden Zellen in dem Zellkulturvolumen dadurch gesteuert werden, dass die Umgebungsgasatmosphäre entsprechend eingestellt wird.

Z. B. wird die Zellkultur-Bestrahlungskammer in einer hypoxischen Umgebungsgasatmosphäre angeordnet. Durch die Diffusion durch die gaspermeable, insbesondere sauerstoffdurchlässige Folie werden die Zellen in dem Zellkulturvolumen hypoxisch. Bezogen auf diese Anwendung kann die Zellkultur-Bestrahlungskammer auch als Hypoxiekammer bezeichnet werden. Vorteilhafterweise ist die Zellkultur-Bestrahlungskammer einfach und kostengünstig herzustellen.

Die Bestrahlung der Zellen in dem Zellkulturvolumen erfolgt durch die vordere der beiden Folien, wobei der Strahl durch die hintere der beiden Folien nahezu ungestört wieder austreten kann, so dass sowohl Streuungen beim Eintritt des Strahls in das Zellkulturvolumen als auch unerwünschte Rückstreueffekte strahlabwärts des Zellkulturvolumens vermieden werden können. Im Folgenden wird der Einfachheit halber die strahlabwärts gelegene Folie als hintere Folie und die strahlaufwärts gelegene Folie als vordere Folie bezeichnet.

Besonders vorteilhaft ist die Erfindung für die Bestrahlung von adhärent wachsenden Zellkulturen. Hierfür weist zumindest eine Folie an ihrer dem Zellkulturvolumen zuwandten Innenseite eine Wachstumsoberfläche zur adhärenten Kultivierung der Zellen auf. Insbesondere ist die vordere Folie gaspermeabel und besitzt innen eine solche Wachstumsoberfläche. Solche Kunststoffolien mit einseitiger Wachstumsoberfläche sind kommerziell erhältlich, z.B. eine Fluorcarbonfolie unter der Bezeichnung lumox^{™} von In Vitro Systems & Services GmbH. Diese Fluorethylen-propylen-Folie mit Wachstumsoberfläche ist etwa 25 µm dick, besitzt eine O₂-Permeabilität von 6,3 µmol/(cm²·h) und eine CO₂-Permeabilität von 2,2 µmol/(cm²·h). Es wird davon ausgegangen, dass die Folie eine Mindestpermeabilität für O₂ von 3,2 µmol/(cm²·h), vorzugsweise 4,5 µmol/(cm²·h), besitzen sollte, um kontrollierte Hypoxiebedingungen zu ermöglichen.

Es erfolgt also durch die gaspermeable Folie ein Gasaustausch zwischen der Umgebungsgasatmosphäre auf der Folienaußenseite unter Verdrängung des Sauerstoffs aus dem Nähr- oder Zellkulturmedium auf der Folieninnenseite. Damit kann ein genau definierter und konstanter Sauerstoffgehalt an der unmittelbar auf der gaspermeablen Folieninnenseite adhärent aufgewachsenen Zellschicht erreicht werden.

Die verwendete Fluorcarbonfolie ist ferner stabil genug und transparent, so dass die auf der hydrophilen Seite der Folie adhärent aufgewachsene Zellkultur nach der Bestrahlung z.B. mit einem Durchlichtmikroskop untersucht werden kann. Dadurch dass der Röntgen- oder Teilchenstrahl lediglich mit den beiden Folien und dem Inhalt des Zellkulturvolumens wechselwirkt, werden unerwünschte Streueffekte so weit wie möglich reduziert. Es wurde ferner keine übermäßige Aktivierung der Folie bei Bestrahlung mit hochenergetischen Kohlenstoffionen festgestellt. Außerdem wurde festgestellt, dass aufgrund der Bestrahlung kein oder kaum für die Hypoxieuntersuchungen störender Sauerstoff aus der Folie austritt und die Ergebnisse verfälscht.

Wenn nur eine Zellkulturschicht unter hypoxischen Bedingungen untersucht werden soll, ist es zweckmäßig die zweite hintere Folie, wenngleich vorzugsweise ebenfalls gaspermeabel, auf der Innenseite nicht mit einer Wachstumsschicht auszustatten, sondern z.B. mit einer hydrophoben Innenseite vorzusehen. Hierdurch wird erreicht, dass die Zellen lediglich an der ersten vorderen Folie adhärent anwachsen. Hierfür kann ggf. dasselbe Folienmaterial wie für die vordere Folie, aber in umgekehrter Orientierung, verwendet werden, so dass mit einer Sorte Folienmaterial ausgekommen werden kann.

Es hat sich bewährt, die beiden Folien mit einer nicht-aushärtenden Klebemasse, z.B. Silikonfett beidseits lösbar auf den Trägerrahmen aufzukleben. Silikonfett hat sich als hinreichend steril erwiesen, so dass das Zellwachstum nicht gestört wird, leistet aber auch die erforderliche Dichtigkeit. Besonders vorteilhaft ist, dass die Folien nach der Verwendung wieder abgezogen werden können und der Trägerrahmen nach einer Reinigung erneut verwendet werden kann. Andere Befestigungsarten, wie z. B. Klemmung, sind jedoch auch denkbar, sofern sie eine hinreichende Dichtigkeit für das Zellkulturmedium gewährleisten.

Vorzugsweise besitzt der Trägerrahmen eine radiale Bohrung oder einen Kanal vom äußeren Rand bzw. von der Schmalseite her bis in das Zellkulturvolumen, so dass durch die Bohrung bzw. den Nährmedium, z.B. mit einer handelsüblichen Spritze und Kanüle in das Zellkulturvolumen zwischen die beiden Folien eingefüllt werden kann. Nach dem Einfüllen wird die Bohrung bzw. der Kanal z. B. mit einer Schraube wieder verschlossen.

Zur Erfindung gehört auch ein geeigneter Begasungscontainer, in den eine oder mehrere Zellkultur-Bestrahlungskammern hintereinander eingesetzt werden können. In dem Begasungscontainer wird dann eine vordefinierte Umgebungsgasatmosphäre um die Zellkultur-Bestrahlungskammer(n) eingestellt, so dass die Gasmoleküle aus der Umgebungsatmosphäre, z. B. N₂ und CO₂, durch die gaspermeable(n) Folie(n) in das Zellkulturmedium und die Zellschicht hinein- und andere Gasmoleküle, insbesondere O₂, herausdiffundieren können.

Die Erfindung betrifft somit auch ein System aus Zellkultur-Bestrahlungskammer und dem Begasungscontainer. Dieses Bestrahlungssystem ist demnach hergerichtet zur Bestrahlung von lebenden Zellen mit einem Röntgenstrahl oder Teilchenstrahl unter definierter Konzentration von bestimmten Gasmolekülen in den Zellen.

Der Begasungscontainer umfasst ein Gehäuse aus einem sterilisierbaren Material, in welchem durch Bestrahlung mit einem hochenergetischen Teilchenstrahl keine langlebigen radioaktiven Isotope erzeugt werden, und in das die Zellkultur-Bestrahlungskammer mit einer vorbereiteten Zellkultur eingesetzt wird. Vorzugsweise umfasst der Begasungscontainer einen einseitig offenen Behälter, der mit einem Deckel lösbar und gasdicht, z. B. mit einem Dichtring, verschlossenen werden kann.

Ferner umfasst der Begasungscontainer einen Gaseinlass zum Einleiten von Prozessgas mit vordefinierter Zusammensetzung und einen Gasauslass, zum Abführen des durch den Gaseinlass in den Begasungscontainer eingeleiteten Prozessgases, so dass der Begasungscontainer mit einem beliebigen Gas von vordefinierter Zusammensetzung gespült werden kann.

In dem geschlossenem Zustand läßt sich demnach eine vordefinierte Begasungsatmosphäre mit kontrollierbarer Zusammensetzung um die Zellkultur-Bestrahlungskammer einstellen. Vorzugsweise hat die Begasungsatmosphäre Kontakt zu beiden Folien mit einer Diffusionsfläche von zumindest einigen cm², so dass die Diffusionsrate in das Zellkulturvolumen hinreichend groß ist. Wird eine sauerstofffreie oder -arme Begasungsatmosphäre in dem Begasungscontainer eingestellt, kann durch die Diffusion durch die gaspermeable (n) Folie (n) aus dem Zellkulturvolumen der Zellkultur-Bestrahlungskammer der Sauerstoff herausdiffundieren, um hypoxische Bedingungen einzustellen. Mit anderen Worten trennt die gaspermeable Folie die Begasungsatmosphäre in dem Begasungscontainer unmittelbar von der adhärent.auf der Folie aufgewachsenen Zellkultur und dem flüssigen Zellkulturmedium.

Der Begasungscontainer weist ferner ein gasdichtes und strahlungsdurchlässiges vorderes Strahleintrittsfenster auf, durch welches der Röntgenstrahl oder Teilchenstrahl in den Begasungscontainer eintreten kann, wobei die Zellkultur-Bestrahlungskammer derart strahlabwärts des Strahleintrittsfensters positioniert ist, dass der Strahl nach dem Durchtritt durch das Strahleintrittsfenster durch die vordere Folie in das Zellkulturvolumen eintritt, dort mit den Zellen wechselwirkt und durch die hintere Folie wieder austritt, wenn der mit der Zellkultur-Bestrahlungskammer bestückte Begasungscontainer mit dem Röntgenstrahl oder Teilchenstrahl bestrahlt wird. Die Zellkultur-Bestrahlungskammer ist in dem Begasungscontainer festgelegt. Mit anderen Worten liegt die Zellkultur bei der Bestrahlung in dem Strahlengang, des durch das Eintrittsfenster gerichteten Röntgen- oder Teilchenstrahls.

Gemäß einer bevorzugten Ausführungsform ist in dem Begasungscontainer ein Stapel aus einer Mehrzahl von Zellkultur-Bestrahlungskammern hintereinander festlegbar, so dass der Röntgenstrahl oder Teilchenstrahl nacheinander jeweils durch die ersten und zweiten Folien aller Zellkultur-Bestrahlungskammern hindurchtritt, um die Zellkulturen in den Zellkulturvolumina aller Zellkultur-Bestrahlungskammern hintereinander zu bestrahlen. Mit einer solchen Anordnung kann z.B. die Bestrahlung eines ausgedehnten Tumors in einem Schritt simuliert werden.

Es wurde herausgefunden, dass sich Polyetherketon, insbesondere Polyetheretherketon (einschließlich Abkömmlingen) besonders als Material für den Behälter eignet. Diese Materialien sind hinreichend stabil, sterilisierbar, insbesondere autoklavierbar und alkoholbeständig. Ferner sind sie aber auch mit Ionenbestrahlung ausreichend nicht aktivierbar und gasen hinreichend wenig Sauerstoff bei der Bestrahlung aus. Der Deckel kann z. B. aus transparentem PMMA bestehen, da er nicht im Strahlengang liegt.

Vorzugsweise ist der Behälter quaderförmig und wurde in diesem Beispiel aus einem Stück gefräst, um Verbindungsstellen zu vermeiden. Ein Teil der vorderen Seitenwand wurde auf eine Dicke von etwa 1 mm ausgedünnt, so dass hierdurch ein einstückig mit dem Behälter ausgebildetes Strahleintrittsfenster gebildet wird. Falls gewünscht, kann an der Rückseite noch ein gasdichtes und strahlungsdurchlässiges Strahlaustrittsfenster gebildet werden, so dass der Strahl nach der Bestrahlung der Zellkultur in dem Zellkulturvolumen aus diesem Begasungscontainer wieder austreten kann. Dies hat den Vorteil, dass mehrere Begasungscontainer hintereinander bestrahlt werden können und Rückstreuung reduziert wird.

Die Quader-Form hat den Vorteil, dass der Begasungscontainer sowohl für eine Bestrahlung mit einem horizontal verlaufenden Strahl als auch mit einem vertikal verlaufenden Strahl sicher auf einem Tisch aufgestellt werden kann. Dadurch eignet sich das Bestrahlungsssystem sowohl für die Bestrahlung mit einem Ionenstrahl (typischerweise horizontal), als auch mit einem Röntgenstrahl (häufig vertikal).

Vorzugsweise weist der Begasungscontainer jeweils eine Gaskupplung mit einem Ventil für den Gaseinlass und den Gasauslass auf. Besonders einfach ist es, wenn das Ventil in der Kupplung integriert ist und automatisch schließt, wenn die Gasschläuche abgezogen werden. Somit kann der Begasungscontainer hermetisch abgeschlossen werden und es kann in vorteilhafter Weise während der Bestrahlung auf einen Gasdurchfluss verzichtet werden. Es wird lediglich vor der Bestrahlung der Begasungscontainer über einen Mindestzeitraum mit mehrfachem Gasaustausch gespült.

Die Gaskupplungen sind vorzugsweise in gegenüberliegenden Seitenwänden eingebaut und liegen nicht in dem durch das Eintrittsfenster und die zu bestrahlende Zellkultur definierten Strahlengang, um eine Aktivierung zu vermeiden. Gleiches gilt für die Verbindungsmittel, z. B. Schrauben und Muttern oder Klammern zwischen Behälter und Deckel.

Gemäß einer weiter bevorzugten Ausführungsform weist der Begasungscontainer eine Injektionsmembran zum sterilen Einstechen einer Kanüle, z. B. eines Sauerstoffsensors auf, um den Sauerstoffgehalt in dem Begasungscontainer zu messen. Kommerziell erhältliche Injektionsmembranen wiesen auch nach mehrfachem Einstechen und wieder Herausziehen noch eine hinreichende Dichtigkeit auf.

Die Vorgehensweise bei der Zellkultivierung und Bestrahlung ist wie folgt. Zunächst wird der Trägerrahmen mit nur einer Folie beklebt, so dass eine einseitig offene Zellkulturschale entsteht, wobei der Trägerrahmen den Innenraum lateral umschließt und die gaspermeable Folie den Boden der Zellkulturschale bildet. Nachfolgend wird in der Zellkulturschale im flach liegenden Zustand auf der Innenseite (oben) der gaspermeablen Folie eine adhärente Zellkultur in einem Nährmedium kultiviert. Vorzugsweise ist die radiale Bohrung, z.B. mit einem Pfropfen verschlossen damit hier das Nährmedium im liegenden Zustand nicht auslaufen kann. Nach hinreichender Wachstumszeit, z. B. liegend in einem Brutschrank wird das Nährmedium aus der einseitig offenen Zellkulturschale abgeschüttet oder abgesaugt, um nachfolgend die zweite, ggf. ebenfalls gaspermeable Folie auf der gegenüberliegenden Oberseite des Trägerrahmens aufzukleben, so dass aus der einseitig offenen Zellkulturschale die beidseits geschlossene Zellkultur-Bestrahlungskammer entsteht.

Nun wird die Zellkultur-Bestrahlungskammer senkrecht mit der radialen Bohrung nach oben in eine spezielle Halterung gestellt und neues Zellkultur- oder Nährmedium durch die radiale Bohrung in das Zellkulturvolumen zwischen den beiden Folien, z.B. mittels einer Spritze durch die radiale Bohrung eingefüllt, bis das Zellkulturvolumen vollständig ausgefüllt ist. Hierdurch wird das Austrocknen der Zellkultur über die gesamte Folienfläche verhindert, wenn die Zellkultur-Bestrahlungskammer später bei der Bestrahlung senkrecht steht. Der Pfropfen wird hierbei durchstochen und die radiale Bohrung anschließend, z.B. mit einer Schraube wieder verschlossen.

Nun wird die befüllte und dichte Zellkultur-Bestrahlungskammer in den Begasungscontainer eingesetzt, der vorzugsweise Nuten aufweist in denen die Zellkultur-Bestrahlungskammer festgelegt wird. Anschließend wird der Begasungscontainers mit dem Deckel dicht verschlossen der Innenraum des Begasungscontainers über eine Mindestzeitdauer mit einer vordefinierten Gasatmosphäre über die Gaszuleitung und die Gasableitung gespült. Das Spülen kann nach der Mindestzeitdauer beendet und die Gasleitungen entfernt werden. Nun wird der Begasungscontainer auf einen Tisch an dem Bestrahlungsplatz gestellt und bestrahlt, vorzugsweise mit der adhärenten Zellkultur an der Innenseite der vorderen (strahlzugewandten) Seite der Zellkultur-Bestrahlungskammer. Der Strahl durchquert die Zellkultur-Bestrahlungskammer und tritt aus der hinteren Folie wieder aus der Zellkultur-Bestrahlungskammer aus.

In vorteilhafter Weise bildet der Begasungscontainer während der Bestrahlung eine hermetisch geschlossene und autarke Umgebungsatmosphäre für die Zellkultur-Bestrahlungskammer ohne Schlauchanbindung. Es kann alternativ aber auch während der Bestrahlung weiter gespült werden, sofern dies gewünscht ist.

Ferner kann der Begasungscontainer in unterschiedlichen Orientierungen abgestellt werden, da die Zellkultur-Bestrahlungskammer dicht ist, so dass die Zellkultur-Bestrahlungskammer sowohl stehend (für horizontale Bestrahlung) als auch liegend (für vertikale Bestrahlung) bestrahlt werden kann.

In vorteilhafter Weise können nach Beendigung der Bestrahlung und Untersuchung der Zellkultur die beiden Folien wieder von dem Trägerrahmen entfernt werden und der Träger gereinigt und wiederverwendet werden.

Im Folgenden wird die Erfindung anhand von Ausführungsbeispielen und unter Bezugnahme auf die Figuren näher erläutert, wobei gleiche und ähnliche Elemente teilweise mit gleichen Bezugszeichen versehen sind und die Merkmale der verschiedenen Ausführungsbeispiele miteinander kombiniert werden können.

### Kurzbeschreibung der Figuren

Es zeigen:
- Fig. 1: eine teilweise geschnittene Frontalansicht des Trägerrahmens,
- Fig. 2: eine Schnittdarstellung in Ausschnittsvergrößerung des Bereichs A aus Fig. 1,
- Fig. 3: eine Draufsicht von oben auf den Trägerrahmen mit Radialbohrung,
- Fig. 4: eine dreidimensionale Darstellung des Behälters des Begasungscontainers,
- Fig. 5: eine dreidimensionale Darstellung des Deckels des Begasungscontainers,
- Fig. 6: eine dreidimensionale Darstellung des zusammengebauten Begasungscontainers mit Gaskupplungen,
- Fig. 7: eine Frontansicht des zusammengebauten Begasungscontainers,
- Fig. 8: einen horizontalen Querschnitt entlang der Linie A-A in Fig. 7,
- Fig. 9: einen Längsschnitt durch den Begasungscontainer entlang der Linie B-B in Fig. 7 mit eingesetzter Zellkultur-Bestrahlungskammer,
- Fig. 10: eine alternative Ausführungsform der Erfindung im Längsschnitt ähnlich Fig. 9,
- Fig. 11: eine dreidimensionale Darstellung des Befüllungsständers mit Zellkultur- Bestrahlungskammer und
- Fig. 12: eine grafische Darstellung der relativen Dosis als Funktion der Eindringtiefe eines Kohlenstoff- Ionenstrahls.

### Detaillierte Beschreibung der Erfindung

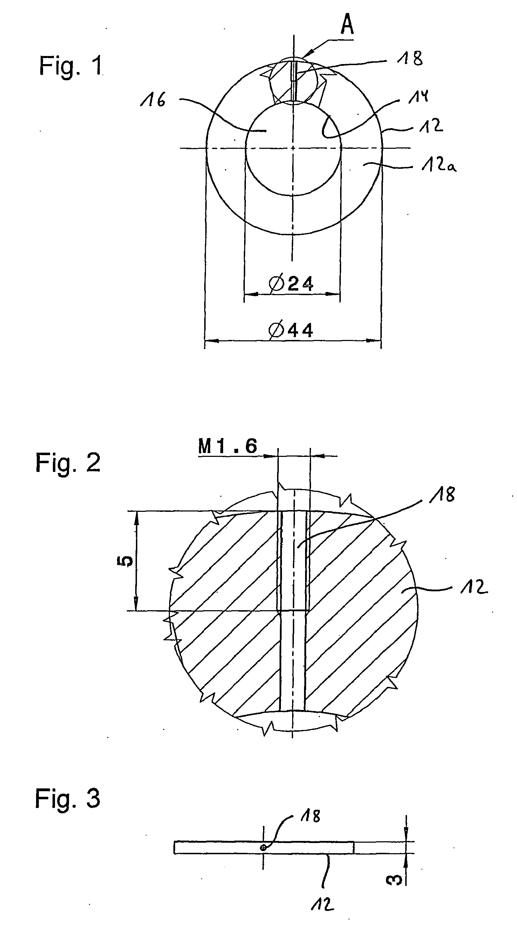

Fig. 1 zeigt den Trägerrahmen 12 der Zellkultur-Bestrahlungskammer 10. Der Trägerrahmen 12 ist in diesem Beispiel ringförmig ausgebildet und umschließt mit einer umlaufenden Ringwandung 14 das Zellkulturvolumen 16. Durch den Trägerrahmen 12 verläuft eine radiale Bohrung 18 zum Befüllen des Zellkulturvolumens mit Nährmedium, wenn das Zellkulturvolumen 16 beidseits mit Folien verschlossen ist. In diesem Beispiel ist der Trägerrahmen oder Ring 12 als Kreisring geformt, es ist jedoch ersichtlich, dass andere Formen ebenfalls möglich sind.

Bezug nehmend auf Fig. 2 weist die radiale Bohrung 18 ein Innengewinde 20 auf, so dass die radiale Bohrung 18 mit einer nicht dargestellten Schraube verschlossen werden kann. Die radiale Bohrung 18 erstreckt sich vollständig durch den Trägerrahmen 12.

Der Trägerrahmen 12 besteht aus einem Hart-PVC-Ring mit einem Außendurchmesser von 44 mm, einem Innendurchmesser von 24 mm und einer Dicke von 3 mm. Die radiale Bohrung hat einen Durchmesser von 1,6 mm.

Für die Kultivierung der Zellen wurde eine gaspermeable Folie von der Firma In Vitro Systems & Services unter dem Handelsnamen "Biofolie25" verwendet. Die Biofolie25 ist eine Fluorethylenpropylen-Folie von 25 µm Dicke. Eine Seite ist hydrophil und daher für die Kultivierung adhärent wachsender Zellen geeignet. Die andere Seite der Folie ist hydrophob und daher auch für Suspensionskulturen geeignet. Die Biofolie25 ist gaspermeabel mit folgenden Werten: CO₂: 2,2 µmol/(cm²·h) und O₂: 6,3 µmol/(cm²·h). Aus der Folie, die in Bahnen von 5 m x 305 mm geliefert wird, werden Kreise mit dem Außendurchmesser des Trägerrahmens 12 geschnitten.

Zunächst wird ein Kreis aus Biofolie25 mit Silikonpaste auf den die erste Seite 12a des PVC-Rings 12 geklebt. Auch die radiale Bohrung 18 wird mit Silikonpaste abgedichtet. Die hydrophile Seite der Folie zeigt in Richtung des Zellkulturvolumens oder Innenraums 16. Anschließend wird der Trägerrahmen 12 mit dieser ersten angeklebten Folie 50a nach unten auf eine Unterlage gelegt, so dass die Anordnung eine Zellkulturschale, ähnlich einer Petrischale bildet, wobei die gaspermeable erste Folie 50a den Schalenboden bildet. Die Zellen werden dann in der so entstandenen Zellkulturschale kultiviert und anschließend das Nährmedium entfernt, z. B. abgesaugt oder abgeschüttet. Anschließend wird auf die zweite Seite des PVC-Rings 12 eine zweite Biofolie25 50b, diesmal mit der hydrophoben Seite nach innen, ebenfalls mit Silikonpaste aufgeklebt. Dann wird durch die radiale Bohrung 18 ein neues Nährmedium eingefüllt und die radiale Bohrung mit einer Schraube aus Polyetheretherketon (PEEK) steril verschlossen. Somit entsteht eine flüssigkeitsdicht geschlossene Kammer. Besonders zu beachten ist, dass das Zellkulturvolumen mit dem Nährmedium oder der Nährlösung selbst nicht begast werden braucht, da die Diffusion der Gasmoleküle durch die Biofolie25 stattfinden kann, wobei die Zellen unmittelbar an der Grenze zwischen Flüssigphase im Zellkulturvolumen 16 und Gasphase außerhalb der Zellkultur-Bestrahlungskammer 10 gewachsen sind.

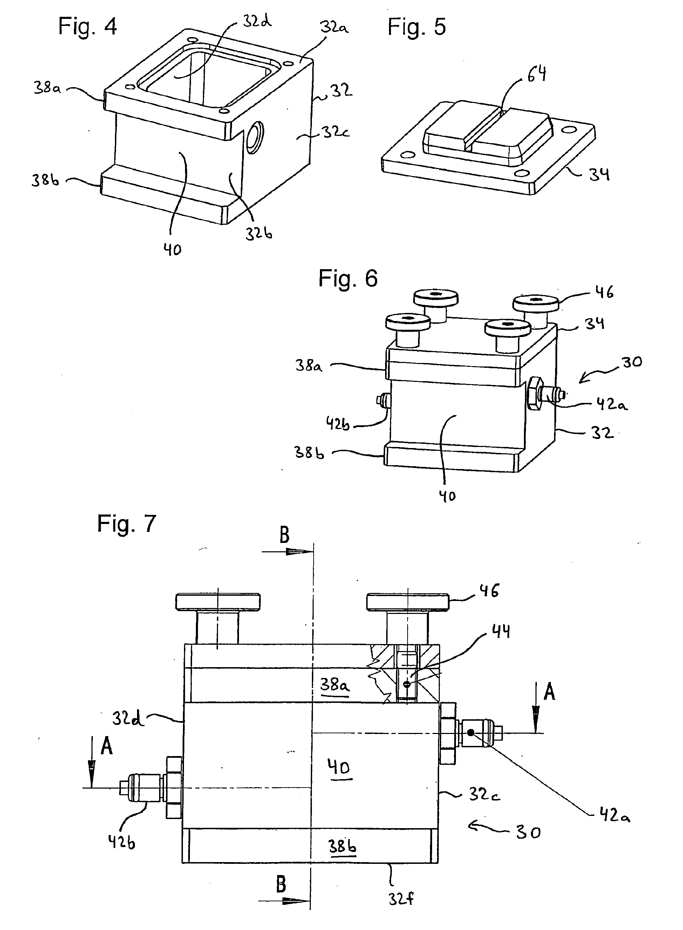

Bezug nehmend auf die Fig. 4 bis 9 ist der Begasungscontainer 30, bestehend aus einem Behälter 32 und einem Deckel 34, dargestellt. Der Begasungscontainer 30 ist im Wesentlichen quaderförmig, so dass er sowohl für die vertikale Bestrahlung in einer Röntgenröhre als auch für die horizontale Bestrahlung mit einem Ionenstrahl geeignet ist, wobei die Zellkultur-Bestrahlungskammer 10 und somit auch die adhärente Zellkulturschicht bei der Bestrahlung vertikal stehen.

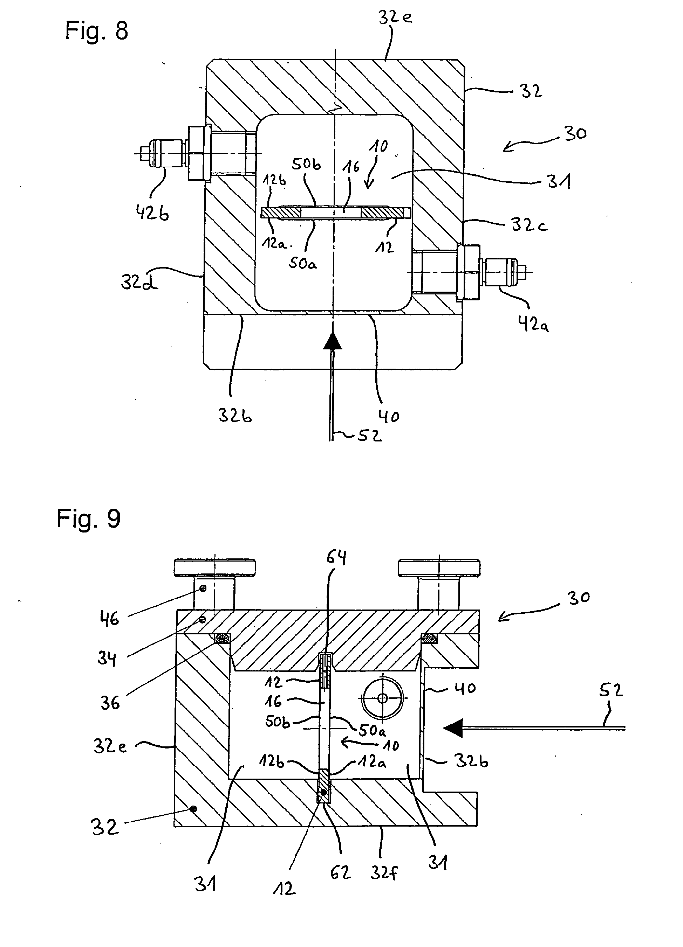

Der Korpus oder Behälter 32 des Begasungscontainers 30 wurde aus einem Stück PEEK (Dichte 1,32 g/cm³) gefräst. PEEK hat den Vorteil, dass es gegenüber hohen Temperaturen und einer Reihe von Chemikalien beständig ist, so dass eine Sterilisation im Autoklaven oder mit 70%igem Ethanol möglich ist. Der Deckel 34 ist aus PMMA gefertigt und daher transparent. Der Behälter 32 weist an seiner offenen Oberseite 32a eine umlaufende Nut für einen O-Ring 36 (vgl. Fig. 9) auf. Die Frontseite 32b ist zwischen zwei Stabilisierungsstegen 38a, 38b auf eine Dicke von 1 mm abgefräst, so dass durch die Ausdünnung ein Strahleintrittsfenster 40 an der Frontseite 32b gebildet wird. An den beiden Lateralseiten 32c, 32d ist jeweils eine Bohrung für eine Gaskupplung 42a bzw. 42b vorgesehen.

Bezug nehmend auf Fig. 6 werden der Behälter 32 und der Deckel 34 mit vier Schrauben 44 und Rändelmuttern 46 lösbar miteinander verbunden. Die Schrauben 44 und Rändelmuttern 46 sind in den Ecken des Begasungscontainers angeordnet, um nicht in den Strahlengang zu geraten.

Bezug nehmend auf Fig. 8-10 ist die Zellkultur-Bestrahlungskammer 10, im Wesentlichen bestehend aus dem Trägerrahmen 12 und beidseits aufgeklebter gaspermeabler Folie 50a, 50b, innerhalb des Begasungscontainers 30 dargestellt. Das Zellkulturvolumen 16 ist mit Nährmedium vollständig gefüllt, und die Zellkultur ist adhärent an der vorderen Folie 50a angewachsen. Der Pfeil 52 repräsentiert den Röntgen- oder Ionenstrahl, mit dem die Zellkultur bestrahlt wird.

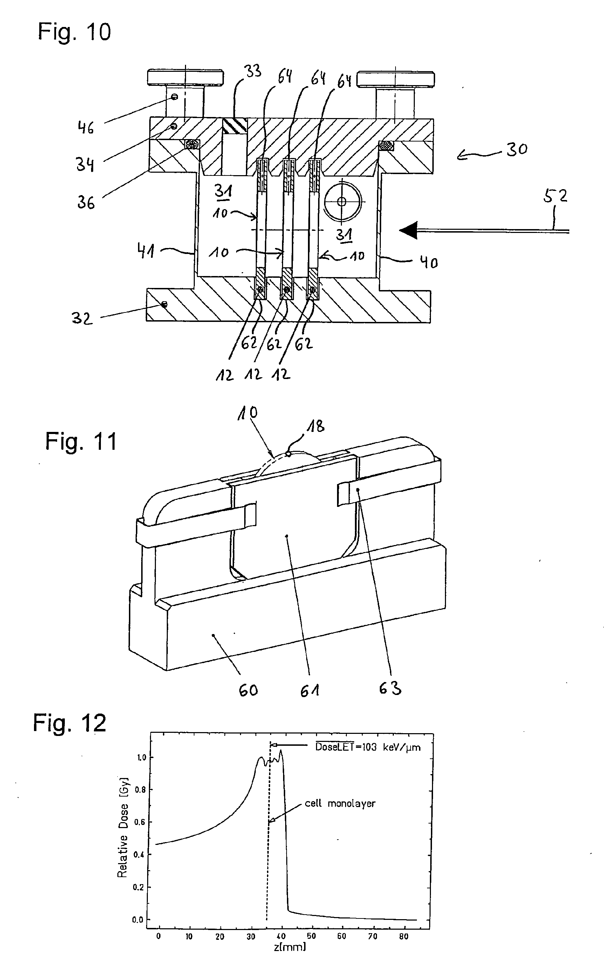

Der Röntgen- oder Ionenstrahl 52 dringt zunächst durch das Strahleintrittsfenster 40 in den Innenraum 31 des Begasungscontainers 30, welcher die Begasungsatmosphäre definiert. Anschließend durchquert der Strahl 52 die vordere gaspermeable Folie 50a, wechselwirkt dann mit der Zellkultur an der Innenseite der vorderen Folie 50a, durchquert das Nährmedium im Zellkulturvolumen 16 und tritt über die hintere, ebenfalls gaspermeable Folie 50b wieder aus dem Zellkulturvolumen 16 aus. Die Ausführungsform des Begasungscontainers 30 in Fig. 8 und 9 besitzt lediglich ein Strahleintrittsfenster 40. Fig. 10 zeigt eine Ausführungsform mit einem Strahlaustrittsfenster 41 und einem Stack mit drei Zellkultur-Bestrahlungskammern 10, die die Bestrahlung in einem sog. "extended volume" sowie von zwei gegenüberliegenden Seiten ermöglicht.

Bezug nehmend auf Fig. 9 und 10 ist im Boden 32f sowie im Deckel 34 jeweils eine Nut 62 bzw. 64 vorgesehen, so dass die Zellkultur-Bestrahlungskammer 10 in dem geschlossenen Begasungscontainer 30 sicher festgelegt ist. Dadurch kann die Bestrahlung horizontal (der Boden 32f steht auf einem Tisch auf) und vertikal (der Begasungscontainer 30 steht mit der Rückwand 32e auf einem Tisch auf) erfolgen. In beiden Fällen stehen die Zellkultur und das Zellkulturmedium in unmittelbarem Kontakt zur vorderen Folie 50a, und die Gasmoleküldiffusion zwischen der Gasatmosphäre 31 im Begasungscontainer 30 und dem flüssigkeitsgefüllten Zellkulturvolumen 16 kann stattfinden.

Bezug nehmend auf Fig. 8 sind die beiden Gaskupplungen 42a, 42b versetzt angeordnet, so dass das Gas dem Begasungscontainer im Durchfluss gleichmäßig und vollständig durchströmt und so ein vollständiger Austausch der Gasatmosphäre im Innenraum 31 erfolgen kann. Der Gasfluss wird mit einem thermischen Massendurchflussmesser direkt vor dem Gaseingang in den Begasungscontainer gemessen.

Zur Messung des Sauerstoffgehalts im Innenraum 31 des Begasungscontainers 30 besitzt dieser gegebenenfalls noch eine Injektionsmembran 33, z. B. im Deckel 34 (vgl. Fig. 10). Durch diese Injektionsmembran 33 kann ein entsprechender Sauerstoffsensor in den Begasungscontainer 30 eingestochen werden, und der Sauerstoffgehalt, z. B. in Prozent Luftsättigung, gemessen werden.

Vor der Kultivierung der Zellen in der Zellkultur-Bestrahlungskammer 10 werden die Folienstücke 50a, 50b und der Trägerrahmen 12 für 15 Minuten in 70%igem Ethanol sterilisiert und anschließend getrocknet. Daraufhin wurde eine Seite des Trägerrahmens 12 mit Silikonpaste (Hersteller: Gehe oder Wacker) flächig eingestrichen und die erste der beiden Folien 50a passgenau aufgedrückt, wobei die hydrophile Seite der Folie 50a in Richtung des Zellkulturvolumens 16, also nach innen, weist, so dass dort Zellen anwachsen können.

Etwa 24 Stunden vor der Bestrahlung wurden in einem ersten Experiment 5 x 10⁴ CHO-K1-Zellen und 1,5 ml Nähr- oder Kulturmedium in die Zellkulturschale 12/50a gegeben und bei 37°C und 5 % CO₂ liegend inkubiert.

Anschließend wird das Nährmedium aus der Zellkulturschale 12/50a abgeschüttet, und Reste des Nährmediums auf dem Rand der der ersten Folie 50a gegenüber liegenden Seite 12b des Trägerrahmens 12 werden mit einem sterilen Wattestäbchen entfernt. Dann wird der Rand der zweiten Seite 12b des Trägerrahmens 12 mit Silikonpaste bestrichen und die zweite gaspermeable Folie 50b mit der hydrophoben Seite nach innen in Richtung des Zellkulturvolumens 16 und den auf der Innenseite der ersten Folie 50a hin aufgeklebt. Um einen gleichmäßigen Anpressdruck zu erreichen, kann hierfür der Deckel einer Petrischale verwendet werden. Dadurch entsteht das beidseits beschlossene Zellkulturvolumen 16.

Bezug nehmend au Fig. 11 wird die Zellkultur-Bestrahlungskammer 10 nachfolgend in einen Befüllungs-Ständer 60 gestellt, wobei die radiale Bohrung 18 nach oben weist. Nun wird frisches Nährmedium in eine Spritze aufgezogen und mit einer Kanüle durch die radiale Bohrung 18 in das Zellkulturvolumen 16 eingespritzt. In dem Ausführungsbeispiel wurden ca. 1,35 ml injiziert, was dem Gesamtvolumen des Zellkulturvolumens 16 entspricht, so dass dieses möglichst vollständig und ohne Luftblase gefüllt wird. Allerdings sollte darauf geachtet werden, dass sich die Folien 50a und 50b nicht wölben. Letzteres ist insbesondere bei der Bestrahlung mehrerer Zellkultur-Bestrahlungskammern 10 hintereinander in einem sogenannten "extended volume" (vgl. Fig. 10) wichtig, da die Dicke der Schicht des Nährmediums bei Bestrahlung mit Ionen die. Strahlenergie für die strahlabwärts gelegenen Zellkultur-Bestrahlungskammern 10 reduziert. Hierfür hat der Ständer 60 zwei parallele transparente und stabile Deckplatten 61, zwischen denen die Zellkultur-Bestrahlungskammer 10 mittels Klammern 63 so eingeklemmt wird, so dass sich die Folien 50a und 50b beim Befüllen nicht nach außen wölben können. Ein weiterer Vorteil des Ständers 60 ist, dass die Gefahr, mit der Kanüle abzurutschen und sich zu verletzen, verringert wird. Nach der vollständigen Befüllung des Zellkulturvolumens 16 mit den Nährmedium wird die radiale Bohrung 18 mit einer Kunststoffschraube verschlossen, wobei das in dem Gewinde verbliebene Silikon eine gute Dichtung bewirkt.

Anschließend werden die befüllten Zellkultur-Bestrahlungseinrichtungen 10 bis zur Bestrahlung in einen Inkubator gestellt.

Alternativ wurden in einem zweiten Experiment R-AT-1 Zellen kultiviert. Die Kultivierung der R-AT-1 Zellen erfolgte analog zu der vorbeschriebenen Kultivierung der CHO-K1 Zellen im liegenden Zustand der einseitig offenen Zellkulturschale 12, 50a. Drei Tage vor der Bestrahlung wurden 2 x 10⁴ R-AT-1 Zellen mit 1 ml Kulturmedium in der Zellkulturschale 12/50a angesetzt. Kurz vor der Bestrahlung wird das Nährmedium mit einer Pipette abgezogen, die zweite gaspermeable Folie 50b aufgeklebt und mit einer Spritze frisches Medium in die Zellkultur- Bestrahlungskammer 10 gefüllt.

In den vorstehend beschriebenen Beispielen befindet sich auf der Innenseite der ersten gaspermeablen Folie 50a eine adhärente Zellkulturschicht. Ein Anwachsen auf der zweiten gaspermeablen Folie 50b wird verhindert. Es ist aber ersichtlich, dass es auch möglich ist, auf den Innenseiten beider Folien 50a, 50b Zellen adhärent zu kultivieren, so dass zwei Zellschichten pro Zellkultur-Bestrahlungskammer 10 auf einmal bestrahlt werden können, falls dies gewünscht wird. Alternativ können auch suspendierte Zellkulturen hergestellt und bestrahlt werden.

Bezug nehmend auf die Fig. 8 und 9 wird für die Begasung der Zellkultur-Bestrahlungskammer 10 zunächst der Begasungscontainer 30 mit 70%igem Ethanol sterilisiert. Anschließend wird die befüllte Zellkultur-Bestrahlungskammer 10 mit den kultivierten Zellen aus dem Inkubator genommen und in den Spalt 62 des Behälters 32 gestellt. Die erste Folie 50a, auf der die Zellen kultiviert wurden, zeigt dabei nach vorne in Richtung des Strahleintrittsfensters 40. Nach dem Aufschrauben des Deckels 34 wird die Begasung über die Kupplung 42a und 42b im Durchflussbetrieb gestartet. Für eine Messung der akuten Hypoxie wird der Begasungscontainer 30 bei Raumtemperatur über eine Zeitdauer von 1,5 Stunden begast. Um den in dem Medium innerhalb des Zellkulturvolumens 16 gelösten Sauerstoff zu entfernen und hypoxische Bedingungen einzustellen, wurden zwei Gasgemische, nämlich 5 % CO₂ und 95 % N₂ sowie 0,5 % O₂, 5 % CO₂ und 94,5 % N₂ verwendet.

Für die chronische Hypoxie wurde der Begasungscontainer über eine Zeitdauer von 1,5 Stunden bei einer Durchflussrate von 200 ml/min mit einem Gasgemisch aus 0,5 % O₂, 5 % CO₂ und 94,5 % N₂ begast. Anschließend wurden die Zuleitungsschläuche entfernt und der Begasungscontainer für 22,5 Stunden in einen Inkubator gestellt und die Zellen bei einer Temperatur von 37°C kultiviert. Die Bestrahlung der Zellen erfolgte demnach 24 Stunden nach Beginn der Begasung mit Röntgenstrahlen mit verschiedenen Dosen zwischen 3 und 30 Gy.

Fig. 12 zeigt ein typisches Dosisprofil mit verbreitertem Bragg-Peak für einen Kohlenstoff-Ionenstrahl. Die Energie des Ionenstrahls wird so eingestellt, dass die Zellschicht in dem verbreiterten Bragg-Peak liegt.

Es ist dem Fachmann ersichtlich, dass die vorstehend beschriebenen Ausführungsformen beispielhaft zu verstehen sind, und die Erfindung nicht auf diese beschränkt ist, sondern in vielfältiger Weise variiert werden kann, ohne die Erfindung zu verlassen. Ferner ist ersichtlich, dass die Merkmale unabhängig davon, ob sie in der Beschreibung, den Ansprüchen, den Figuren oder anderweitig offenbart sind auch einzeln wesentliche Bestandteile der Erfindung definieren, selbst wenn sie zusammen mit anderen Merkmalen gemeinsam beschrieben sind.

## Patentansprüche

1. Zellkultur-Bestrahlungskammer (10) zur Bestrahlung von lebenden Zellen mit einem Röntgenstrahl oder Teilchenstrahl (52), insbesondere zum experimentellen Ermitteln der Überlebensrate von hypoxischen Testzellen für die Tumorstrahlentherapie, umfassend:
einen Trägerrahmen (12), der einen Innenraum umschließt,
eine erste Folie (50a), welche auf der ersten Seite (12a) des Trägerrahmens (12) befestigt ist und den Innenraum vollständig überspannt und auf der ersten Seite (12a) des Trägerrahmens (12) flüssigkeitsdicht verschließt,
eine zweite Folie (50b), welche auf der gegenüberliegenden zweiten Seite (12b) des Trägerrahmens (12) befestigt ist und den Innenraum vollständig überspannt und auf der zweiten Seite (12b) des Trägerrahmens (12) flüssigkeitsdicht verschließt,
so dass die beiden Folien (50a, 50b) von dem Trägerrahmen (12) beabstandet gehalten werden und zwischen den beiden Folien ein mit einem Zellkulturmedium befüllbares Zellkulturvolumen (16) gebildet wird, welches beidseits unmittelbar von den beiden Folien (50a, 50b) und von einer inneren Ringwandung (14) des Trägerrahmens (12) begrenzt wird,
wobei zumindest eine der beiden Folien (50a, 50b) eine gaspermeable Folie ist, so dass durch die gaspermeable Folie Gasmoleküle in das Zellkulturvolumen (16) hineindiffundieren und herausdiffundieren können, um den Gehalt vorbestimmter Gasmoleküle in den lebenden Zellen in dem Zellkulturvolumen (16) zu steuern.

2. Zellkultur-Bestrahlungskammer (10) nach Anspruch 1, wobei der Trägerrahmen (12) einen Kanal (18) aufweist, welcher vom äußeren Rand des Trägerrahmens (12) in das Zellkulturvolumen führt, wobei der Kanal (18) vom äußeren Rand des Trägerrahmens (12) her zugänglich ist, so dass Zellkulturmedium von dem äußeren Rand her mit einer Kanüle durch den Kanal (18) in das Zellkulturvolumen (16) zwischen die beiden Folien injiziert werden kann.

3. Zellkultur-Bestrahlungskammer (10) nach Anspruch 1 oder 2, wobei die gaspermeable erste Folie (50a) an ihrer dem Zellkulturvolumen (16) zuwandten Innenseite eine Wachstumsoberfläche zur adhärenten Kultivierung der Zellen aufweist, so dass das Umgebungsgas, welches durch die erste Folie hindurchdiffundiert, unmittelbar mit den adhärent auf der Innenseite der gaspermeablen ersten Folie aufgewachsenen Zellen in Wechselwirkung tritt.

4. Zellkultur-Bestrahlungskammer (10) nach Anspruch 1, 2 oder 3, wobei die zweite Folie (50b) eine hydrophobe Innenfläche aufweist, um ein adhärentes Zellwachstum an der zweiten Folie (50b) zu verhindern.

5. Zellkultur-Bestrahluhgskammer (10) nach einem der vorstehenden Ansprüche, wobei die gaspermeable erste Folie (50a) eine Sauerstoffpermeabilität von zumindest 3,2 µmol/(cm²·h) aufweist.

6. Zellkultur-Bestrahlungskammer (10) nach einem der vorstehenden Ansprüche, wobei beide Folien (50a, 50b) gaspermeabel sind.

7. Zellkultur-Bestrahlungskammer (10) nach einem der vorstehenden Ansprüche, wobei die beiden Folien (50a, 50b) mit einer nicht-aushärtenden Klebemasse beidseits lösbar auf den Trägerrahmen (12) aufgeklebt sind.

8. Zellkultur-Bestrahlungskammer (10) nach einem der vorstehenden Ansprüche, wobei die gaspermeable Folie eine transparente Fluorcarbonfolie ist.

9. Bestrahlungssystem zur Bestrahlung von lebenden Zellen mit einem Röntgenstrahl oder Teilchenstrahl unter definierter Konzentration von bestimmten Gasmolekülen in den Zellen, umfassend einen Begasungscontainer (30) und zumindest eine Zellkultur-Bestrahlungskammer (10), insbesondere nach einem der vorstehenden Ansprüche, wobei der Begasungscontainer (30) Folgendes umfasst:
ein Gehäuse (32, 34) in das die Zellkultur-Bestrahlungskammer (10) mit einer vorbereiteten Zellkultur einsetzbar ist, wobei das Gehäuse in geschlossenem Zustand eine Begasungsatmosphäre mit kontrollierbarer Zusammensetzung um die Zellkultur-Bestrahlungskammer (10) bildet, so dass Gasmoleküle aus der Begasungsatmosphäre in dem Begasungscontainer (30) in das Zellkulturvolumen (16) der Zellkultur-Bestrahlungskammer (10) hineindiffundieren können und umgekehrt,
einen Gaseinlass (42a) zum Einleiten von Prozessgas mit vordefinierter Zusammensetzung in den Begasungscontainer (30),
einen Gasauslass (42a), zum Abführen des durch den Gaseinlass in den Begasungscontainer (30) eingeleiteten Prozessgases,
ein Strahleintrittsfenster (40), durch welches der Röntgenstrahl oder Teilchenstrahl (52) in den Begasungscontainer (30) eintreten kann, wobei die Zellkultur-Bestrahlungskammer (10) derart strahlabwärts des Strahleintrittsfensters (40) positioniert ist, dass der Strahl (52) nach dem Durchtritt durch das Strahleintrittsfenster (40) durch eine vordere Folie (50a) in das Zellkulturvolumen (16) eintritt, dort mit den Zellen wechselwirkt und durch eine hintere Folie (50b) wieder austritt, wenn der mit der Zellkultur-Bestrahlungskammer (10) bestückte Begasungscontainer (30) mit dem Röntgenstrahl oder Teilchenstrahl (52) bestrahlt wird.

10. Bestrahlungssystem nach Anspruch 9, wobei ein Stapel aus einer Mehrzahl von Zellkultur-Bestrahlungskammern (10) hintereinander in dem Begasungscontainer (30) angeordnet ist, so dass der Röntgenstrahl oder Teilchenstrahl (52) nacheinander jeweils durch die ersten und zweiten Folien (50a, 50b) aller Zellkultur-Bestrahlungskammern (10) hindurchtritt, um die Zellkulturen in den Zellkulturvolumina (16) aller Zellkultur-Bestrahlungskammern (10) zu bestrahlen.

11. Bestrahlungssystem nach Anspruch 9 oder 10, wobei das Strahleintrittsfenster (40) einstückig mit einer Seitenwand des Begasungscontainers (30) ausgebildet ist.

12. Bestrahlungssystem nach einem der vorstehenden Ansprüche, wobei das Gehäuse zumindest zweiteilig aus einem Behälterteil (32)und einem Deckelteil (34) ausgebildet ist und Verbindungsmittel (44, 46) zum lösbaren Verbinden des Behälterteils (32) mit dem Deckelteil (34) und Dichtungsmittel (36) zwischen dem Behälterteil (32) und dem Deckelteil (34) aufweist, um das Behälterteil (32) und das Deckelteil (34) gasdicht miteinander zu verbinden, so dass in dem verbundenen Zustand ein geschlossenes inneres Begasungsvolumen (31) definiert wird.

13. Bestrahlungssystem nach Anspruch 12, wobei das Behälterteil (32) einen Boden (32f) und mit dem Boden einstückig ausgebildete Seitenwände (32b-e) aufweist und das Strahleintrittsfenster (40) in einer der Seitenwände (32b) angeordnet ist, so dass der Teilchenstrahl (52) horizontal in das Begasungsvolumen (31) eintreten kann, wenn der Behälter mit dem Boden (32f) auf einer Unterlage steht und wobei das Behälterteil (32) einstückig ausgebildet ist und das Strahleintrittsfenster (40) als ein ausgedünnter Bereich der vorderen Seitenwand (32b) ausgebildet ist.

14. Bestrahlungssystem nach Anspruch 12 oder 13, wobei das Behälterteil (32) aus einem Polyetherketon besteht.

15. Bestrahlungssystem nach einem der vorstehenden Ansprüche, wobei das Gehäuse (32, 34) im Wesentlichen quaderförmig ausgebildet ist, so dass die Zellkultur-Bestrahlungskammer (10) zur horizontalen und vertikalen Bestrahlung geeignet ist.

16. Bestrahlungssystem nach einem der vorstehenden Ansprüche, wobei der Gaseinlass (42a) und der Gasauslass (42b) jeweils eine Gaskupplung mit einem Ventil aufweist, so dass der Begasungscontainer (30) hermetisch abgeschlossen ist, wenn die Gasschläuche von den Gaskupplungen getrennt sind und der Begasungscontainer (30) in hermetisch abgeschlossene Zustand ohne Gasschläuche und ohne Gasdurchfluss bestrahlt werden kann.

17. Bestrahlungssystem nach einem der vorstehenden Ansprüche, wobei gegenüber dem Strahleintrittsfenster (40) ein Strahlaustrittsfenster vorgesehen ist, wobei das Zellkulturvolumen (16) der Zellkultur-Bestrahlungskammer (10) im Strahlengang zwischen dem Strahleintrittsfenster (40) und dem Strahlaustrittsfenster angeordnet ist, so dass der Strahl (52) nach der Bestrahlung der Zellkultur in dem Zellkulturvolumen (16) aus diesem Begasungscontainer (30) austreten kann und zur Bestrahlung einem weiteren Begasungscontainer (30) zugeführt werden kann.

18. Bestrahlungssystem nach einem der vorstehenden Ansprüche, wobei das Gehäuse (32, 34) eine Injektionsmembran (33) zum sterilen Einstechen einer Kanüle aufweist.

19. Bestrahlungssystem zur Bestrahlung von lebenden Zellen mit einem Röntgenstrahl oder Teilchenstrahl unter definierter Konzentration von bestimmten Gasmolekülen in den Zellen, umfassend einen Begasungscontainer (30) und zumindest eine Zellkultur-Bestrahlungskammer (10),
wobei die Zellkultur-Bestrahlungskammer (10) einen Trägerrahmen (12) und eine erste und zweite Folie (50a, 50b) aufweist,
wobei der Trägerrahmen (12) einen Innenraum umschließt und die beiden Folien (50a, 50b) auf gegenüberliegenden Seiten (12a, 12b) des Trägerrahmens (12) befestigt sind und von dem Trägerrahmen (12) beabstandet gehalten werden, wobei die beiden Folien (50a, 50b) den Innenraum überspannen, so dass zwischen den beiden Folien (50a, 50b) ein mit einem Zellkulturmedium befüllbares Zellkulturvolumen (16) gebildet wird, welches beidseits von den beiden Folien (50a, 50b) und von einer inneren Ringwandung (14) des Trägerrahmens (12) begrenzt wird, und
wobei zumindest eine der beiden Folien (50a, 50b) eine gaspermeable Folie ist,
wobei der Begasungscontainer (30) Folgendes umfasst:
ein Gehäuse (32, 34) in das die Zellkultur-Bestrahlungskammer (10) mit einer vorbereiteten Zellkultur einsetzbar ist, wobei das Gehäuse in geschlossenem Zustand eine Begasungsatmosphäre mit kontrollierbarer Zusammensetzung um die Zellkultur-Bestrahlungskammer (10) bildet, so dass Gasmoleküle aus der Begasungsatmosphäre in dem Begasungscontainer (30) durch die gaspermeable Folie (50a, 50b) in das Zellkulturvolumen (16) der Zellkultur-Bestrahlungskammer (10) hineindiffundieren können und umgekehrt,
einen Gaseinlass (42a) zum Einleiten von Prozessgas mit vordefinierter Zusammensetzung in den Begasungscontainer (30),
einen Gasauslass (42a), zum Abführen des durch den Gaseinlass in den Begasungscontainer (30) eingeleiteten Prozessgases,
ein Strahleintrittsfenster (40), durch welches der Röntgenstrahl oder Teilchenstrahl (52) in den Begasungscontainer (30) eintreten kann, wobei die Zellkultur-Bestrahlungskammer (10) derart strahlabwärts des Strahleintrittsfensters (40) positioniert ist, dass der Strahl (52) nach dem Durchtritt durch das Strahleintrittsfenster (40) durch die vordere Folie (50a) in das Zellkulturvolumen (16) eintritt, dort mit den Zellen wechselwirkt und durch die hintere Folie (50b) wieder austritt, wenn der mit der Zellkultur-Bestrahlungskammer (10) bestückte Begasungscontainer (30) mit dem Röntgenstrahl oder Teilchenstrahl (52) bestrahlt wird.

20. Verfahren zum Bestrahlen von biologischen Zellen zumindest mit folgenden Schritten:
(a) Bereitstellen einer einseitig offenen Zellkulturschale (12, 50a) mit einem Trägerrahmen (12), der einen Innenraum umschließt, und einer gaspermeablen ersten Folie (50a) mit einer Wachstumsoberfläche auf der Innenseite, wobei die gaspermeablen erste Folie (50a) an der Unterseite (12a) des Trägerrahmens (12) flüssigkeitsdicht befestigt ist und den Innenraum vollständig überspannt, so dass eine nach oben offene Schale gebildet wird, wobei der Boden aus der gaspermeablen erste Folie (50a) besteht,
(b) Kultivieren einer adhärenten Zellkultur auf der gaspermeablen ersten Folie (50a) in einem Zellkulturmedium in der einseitig offenen Zellkulturschale (12, 50a) aus Schritt (a),
(c) Entfernen des Zellkulturmediums aus der einseitig offenen Zellkulturschale (12, 50a),
(d) Aufbringen einer zweiten Folie (50b) auf der gegenüberliegenden Oberseite (12b) des Trägerrahmens (12), wobei der Innenraum vollständig überspannt wird und der Innenraum auf der Oberseite (12b) des Trägerrahmens (12) flüssigkeitsdicht verschlossen wird und aus der einseitig offenen Zellkulturschale (12, 50a) eine Zellkultur-Bestrahlungskammer (10) mit beidseits geschlossenem Zellkulturvolumen (16) entsteht,
(e) Befüllen des Zellkulturvolumens (16) mit neuem Zellkulturmedium durch eine radiale Bohrung (18) in dem Trägerrahmen (12), wobei die beidseits geschlossene Zellkultur-Bestrahlungskammer (10) aufrecht steht, so dass die radiale Bohrung (18) nach oben weist und nachfolgend Verschließen der Bohrung,
(f) Einsetzen der Zellkultur-Bestrahlungskammer (10) in einen Begasungscontainer (30) mit einem Strahleintrittsfenster (40),
(g) Schließen des Begasungscontainers (30) und Spülen des Innenraums (31) des Begasungscontainers (30) mit einer vordefinierten Gasatmosphäre,
(h) Bestrahlen der Zellkultur in der Zellkultur-Bestrahlungskammer (10) durch das Strahleintrittsfenster (40) und die vordere (50a) der beiden Folien, wobei der Strahl durch die hintere (50b) der beiden Folien aus der Zellkultur-Bestrahlungskammer (10) wieder austritt.

21. Verfahren nach Anspruch 20, wobei die vordere Folie (50a) die gaspermeable erste Folie mit der adhärent aufgewachsenen Zellkultur ist.

22. Verfahren nach Anspruch 20 oder 21, wobei nach Beendigung der Bestrahlung und Untersuchung der Zellkultur die beiden Folien (50a, 50b) von dem Trägerrahmen (12) entfernt werden und der Trägerrahmen (12) wiederverwertet wird.
